# EUROPEAN PATENT APPLICATION

(11) **EP 2 631 649 A1**
(43) Date of publication of application: **28.08.2013**
(21) Application number: 11833896.1
(22) Date of filing: 17.10.2011
(51) Int. Cl.: G01N 33/53

(54) **METHOD AND DEVICE FOR THE RAPID DIAGNOSIS OF DISEASES IN FAECAL SAMPLES**

(30) Priority: 19.10.2010 ES 201031537 P
(71) Applicant: Certest Biotec, S.L., 50018 Zaragoza (ES)
(72) Inventor: GENZOR ASÍN, Carlos, E-50018 Zaragoza (ES); LANDETA ELORZ, Oscar, E-50018 Zaragoza (ES); VELASCO MICHELENA, Beatriz, E-50018 Zaragoza (ES)
(74) Representative: Carpintero Lopez, Francisco
(86) International application number: PCT/ES2011/070714
(87) International publication number: WO 2012/052586

(57) **Abstract**

A rapid diagnostic method and device which, by the application of a sample of faeces dispersed in a dluent, allows the simultaneous detection and differentiation of faecal lactoferrin and calprotectin to be performed using the formation of conjugates with coloured particles and their uptake onto a porous membrane.

## Description

### Technical field of the invention

The present invention lies within the area of biotechnology and in particular the diagnosis of gastrointestinal diseases. The purpose of the invention is to provide a rapid and simple procedure for the detection of faecal lactoferrin (hLf) and calprotectin (hCp) as markers for the diagnosis of inflammatory bowel disease (IBD), infectious diarrhoea (ID) and colorectal cancer (CRC).

### State of the art

Inflammatory bowel disease.

Inflammatory bowel disease (IBD) is a general term used to describe certain diseases that are characterised by an idiopathic and chronic inflammation of the digestive tract. Inflammatory bowel diseases (IBD) include Crohn's disease (CD), ulcerative colitis (UC) and a non-specific or indeterminate type of colitis (IC).

On many occasions and due to the fact that their symptoms are very similar, IBD is very difficult to distinguish from irritable bowel syndrome (IBS). The latter is a benign disease, as opposed to IBD, but the symptoms are very similar to those of IBD.

Inflammatory processes such as IBD are usually associated with the presence of leukocytes. For a reliable diagnosis of the disease a variety of possible markers have been identified.

In the European patent EP0641441 an in vitro test is described for leukocyte determination in stool samples using an immunoassay with anti-lactoferrin antibodies. The patent US7192724 has already described a method for differentiating IBD from IBS by measuring the faecal lactoferrin concentration using an ELISA immunoassay. In fact, it has been established that knowledge of this concentration can be useful when monitoring the disease's development (Patent US7560240).

Furthermore, the patent US 5455160 reveals an immunological method for screening for IBD and CRC using an ELISA test to determine calprotectin concentration in stool samples.

Other possible markers have been described for the diagnosis of IBD, amongst which ANCA and ANSA stand out, although the best results are obtained with faecal lactoferrin and calprotectin (Turkay, C. et Kasapoglu, B. CLINICS 2010; 65(2):221-31). Vieria, A. et al (BMC Research Notes 2009, 2:221) demonstrated that the measurement of both faecal lactoferrin and calprotectin by ELISA are highly sensitive and specific markers for IBD and that they correlate with the degree of inflammation. Finally, Gisbert, J. et al. (Inflamm. Bowel Dis. 2009; 15:1190-1198) determined that the separate measurement of both markers is useful in predicting a relapse in patients with CD or UC.

Along with these patents and experimental results, both ELISA tests and immunochromatographic tests (ICT) are available on the market for the separate determination of lactoferrin and calprotectin. LEUKO EZ VUE™ by Techlab (Blacksburg, VA) is an ICT for the qualitative detection of elevated lactoferrin levels in stool samples. Prevent ID Caldetect by Preventis (Bensheim, Germany) is a semi-quantitative ICT for the detection of calprotectin in faeces to differentiate between IBD and IBS, as is the Quantum Blue Calprotectin Rapid Test by Bühlmann (Basilea, Switzerland).

The state of the art describes distinct immunoassays for calprotectin and lactoferrin, i.e. one immunoassay is performed for calprotectin and another for lactoferrin, meaning that separate samples must be prepared, at different dilutions, and different tests carried out, further implying that:
a. More time is required.
b. More manipulations are required along with the inherent risk of error.
c. The results obtained for the two parameters do not directly correlate as they are taken from two different samples.

The purpose of the present invention is to provide a rapid immunochromatographic type test for the simultaneous and differentiated determination of calprotectin and lactoferrin in stool samples which includes the following advantages:
a. Only one sample is prepared. When both proteins are analysed separately there are two different samples in different solvents.
b. They are tested simultaneously and as such, the test time is reduced, as well as the number of manipulations, therefore reducing the risk of error. The simultaneous determination of two markers increases the sensitivity of the test.
c. The determination is differentiated, in other words, one line displays the presence of lactoferrin and another distinct line displays the presence of calprotectin. The differentiated determination of two markers means the test can be more specific.
d. A specific vial is used permitting quantitative sample taking plus an easy and hygienic method of dispersion in the solvent.

Infectious diarrhoea (ID).

Another application of the present invention is in the control of acute diarrhoea. Although the origins of diarrhoea can be varied, it is important to discern whether a non-invasive process (self-limiting, non-inflammatory) or invasive process (aggressive, inflammatory) is being dealt with. Treatment of the former is limited to rehydration and electrolyte replacement therapy, whilst the latter requires a specific treatment, usually with antimicrobial agents.

Diarrhoeas with inflammatory processes are usually caused by *Salmonella, Shigella, Campylobacter jejuni* or *Clostridium difficile.* These are diagnosed by stool culture and identification of the pathogens. Culturing is a lengthy and expensive technique. Choi, S.W. et al. (Journal of Clinical Microbiology, 1996:928-932) proposed the use of latex agglutination as a screening method to detect the presence of lactoferrin in stool samples, thus avoiding culturing in the case of acute diarrhoea.

Shastri, Y.M., et al. (Am. J. Med. 2008, 121(12):1099-106) reported that the determination of faecal calprotectin demonstrated a high correlation with bacterial infections which produce diarrhoea and proposed their determination as a rapid screening method for infectious diarrhoea before attempting culturing.

The patent US6727073 describes an ICT for the diagnosis of infectious diarrhoea which simultaneously detects for lactoferrin plus one or several of the bacterial antigens that can cause the diarrhoea, namely, *Salmonella, E. coli 0157, Listeria, Yersinia, Shigella, Campylobacter jejuni* or *Clostridium difficile.*

The device in the present invention permits the simultaneous detection and differentiation of faecal lactoferrin and calprotectin, and also constitutes a tool in the diagnosis and control of cases of acute diarrhoea.

Colorectal cancer (CRC).

Colorectal cancer is a disease in which the malignant cells are located in the intermediate and longest parts of the large intestine. One of the most used diagnostic tools for the screening of CRC is the detection of faecal occult blood (FOB). The more sensitive and specific method is an immunoassay to detect haemoglobin, whether using an ELISA test or a rapid test (ICT).

Patent US5552292 describes a CRC diagnostic method consisting of an ELISA immunoassay to detect faecal lactoferrin or myeloperoxidase.

The document WO 2009/065551 A1 reveals a CRC diagnostic method derived from stool samples that uses calprotectin and the haemoglobin-haptoglobin complex as markers.

The device in the present invention permits the simultaneous detection and differentiation of faecal lactoferrin and calprotectin, and also constitutes a useful tool in the diagnosis and screening of colorectal cancer (CRC).

### Description of the invention

The diagnostic method of the present invention consists in: taking a representative portion of the stool sample, dispersing it in a specific solvent, applying the sample dispersion to a specific immunochromatographic device, formation of a complex between the antibodies from the device and the antigens from the sample, and the visualisation of the complexes on the device's membrane.

The immunochromatographic device used is of single use. No type of instrumentation is required to carry out the method or interpret the result. Faeces are used as a sample.

The sample preparation time is approximately two minutes and the test's development time is from 5 to 10 minutes, meaning that the test is 10 to 20 times quicker than EIA tests (ELISA), which are also more complicated to perform and require laboratory instruments.

Due to its simplicity, the test can be carried out in the doctor's surgery, or even, with adequate instructions, by the patient themselves.

The method of the invention is useful for the diagnosis of IBD and its differentiation from IBS. The latter is a benign disease in which the symptoms are very similar to those of IBD.

In the case of acute diarrhoea, the method of the invention is useful as a rapid form of determining whether the diarrhoea is of an inflammatory or invasive nature and if it is necessary to proceed to culturing and/or treatment with antimicrobial agents.

In the case of CRC diagnosis, the invention's test is appropriate for establishing early surveillance procedures for the appearance of the disease, especially in high risk groups (age, family history,...).

### Brief description of the drawings

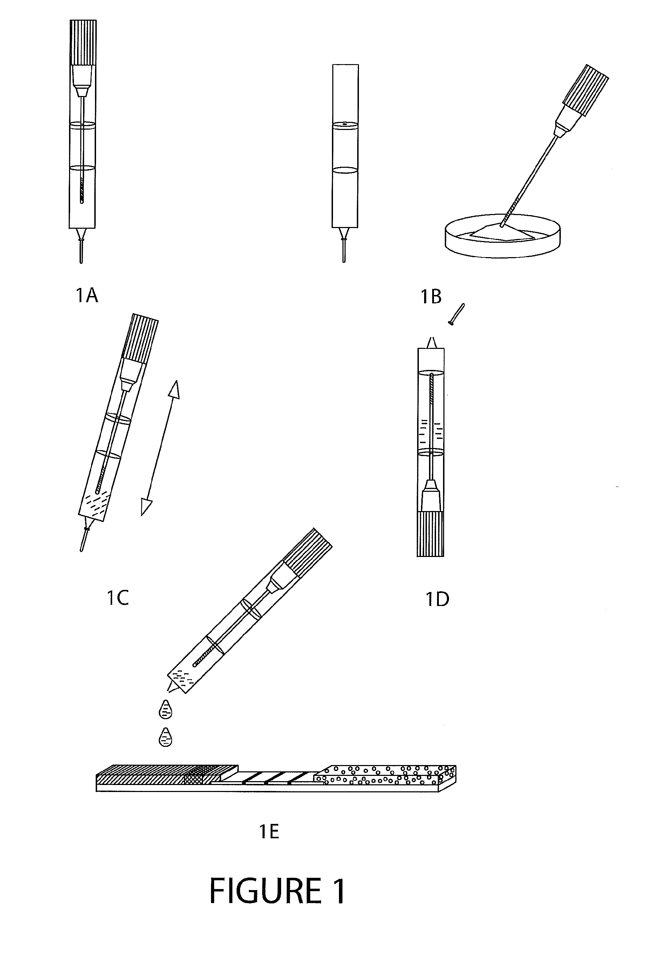

Figure 1: Outline of the stages of the diagnostic method. The specific device or vial for taking stool samples (Figure 1A) is a plastic vial with a capacity of approximately 2 mL, in which 1 mL of solvent has been previously dispensed. The vial has a screw-on lid with a stem whose purpose-designed end permits the quantitative collection of a sample of solid or semi-solid faeces (Figure 1B) and its introduction into the vial. Then, it is closed and vigorously agitated (Figure 1C) until the sample is completely dispersed. Finally, the other end of the device is broken (Figure 1D) and a few drops are placed on the immunochromatographic device (Figure 1E) at the point of sample application.
Figure 2: Views of the immunochromatographic device.
Figure 2A: Top view of the immunochromatographic device in strip format. The said strip includes the following elements: an absorbent material (1), a porous membrane (2) with a control line (3), a line for the detection of calprotectin (4) and another line for the detection of lactoferrin (5), a sample application area (6) and a place for the antibody-marker conjugate (7).
Figure 2B: Side view of the immunochromatographic device in strip format in which the elements described in Figure 2A and the plastic support (8) are identified.
Figure 2C: Projected view of the immunochromatographic device, in which the elements described in Figures 2A and 2B are identified.

### Best method for implementing the invention

lmmunochromatographic devices for the detection of both antigens and antibodies have been described previously (for example, in the patent EP 1248112).

The present invention refers to a three-line test: one for the control, another for lactoferrin detection and the third for calprotectin detection.

In a preferred embodiment, a single anti-calprotectin monoclonal antibody is used. The monoclonal antibody (CA1) has been developed using highly purified calprotectin. The resulting monoclonal antibody displays a high affinity and specificity for the complex formed by the two subunits of calprotectin, whilst it hardly forms any complex at all with either of the individual subunits.

In another preferred embodiment, anti-human lactoferrin polyclonal rabbit antibodies are used. These antibodies have been obtained from rabbits immunised with purified human lactoferrin and subsequently purified by affinity chromatography against the same antigen (purified human lactoferrin).

Conjugates of the above mentioned antibodies are individually prepared with coloured nanoparticles. The nanoparticles are deposited over one of the immunochromatographic devices materials and act as a mobile phase. The antibodies themselves are immobilised separately over the porous membrane, which acts as the stationary phase where the human lactoferrin and calprotectin are immunologically and separately captured. The membrane and the material with the conjugate are mounted, with the aid of other materials, over a plastic support that is cut into a strip.

To perform the test the faeces sample is resuspended in the sample dispersion solvent at a proportion of approximately 1:100. If the sample is liquid, mix 10 µL of sample with 1 mL of solvent. If the sample is solid, separate 10 mg of sample using a spatula and disperse it in 1 mL of solvent. It is very convenient to use the sample collection vial in which the sample is placed with the aid of the stem after which it is closed with the screw-on lid. Then it is vigorously shaken before breaking the upper part of the lid and adding 4 - 5 drops over the sample application point of the immunochromatographic device. The design of the stem is such that upon passing through the vial's reducing orifice it allows a predetermined quantity of the faeces sample to be introduced, in this case 10 mg.

After introducing the sample there is a 10 minute wait before the lines can be seen and the result interpreted. The test is negative if only a single line appears in the results window, the control (for example, green); positive for calprotectin if besides the control there also appears a line in the calprotectin position (for example, blue); positive for lactoferrin if besides the control there also appears a line in the lactoferrin position (for example, red); and positive for both (hLf and hCp) if three lines appear (for example, one green, one red and one blue).

### Methods for performing the invention

### Example 1. Preparation and purification of anti-calprotectin monoclonal antibodies.

The monoclonal antibody CA1 was obtained from the hybridoma of the same name. The hybridoma producer of the monoclonal antibody CA1 was obtained via known protocols according to the cellular fusion method and selection of the obtained clones that was originally described by Kohler and Milstein in 1975 (Köhler G, Milstein C. Continuous cultures of fused cells secreting antibody of predefined specificity. Nature. 1975 Aug 7;256 (5517):495-7).

BALB/c type mice were immunised with purified calprotectin (ProtEra S.r.l., Florence, Italy). The lymphocytes of the immunised mice were fused with myeloma cells from the SP20 line and the obtained hybridomas were selected via ELISA techniques in the following manner: the wells of a 96-well microtitre plate were lined with anti-calprotectin polyclonal rabbit antibodies in 100 mM carbonate buffer at pH 9 and 37 °C for 2 hours. The purified calprotectin was then added to the wells. After washing, different hybridoma culture samples are added and the presence of the anti-calprotectin antibody is revealed by using an IgG anti-m conjugated with peroxide and the corresponding substrate.

The hybridomas with the greatest affinity and best specificity were selected. Amongst them, the most productive and those which offer the best performance in the thermal stress test and in the best rate of reaction in response to the antigen test were selected.

The CA1 hybridoma selected was cultured in RPMI-HT medium for several days at 37 °C with 5% CO₂. The antibody was purified from the culture medium by protein A affinity chromatography according to the instructions of the column manufacturer (GE Healthcare) after which it was dialysed against PBS at pH 7.4.

### Example 2. Preparation and purification of anti-lactoferrin polyclonal antibodies.

The human lactoferrin was purified by ion-exchange chromatography from the commercial product 'Lactoferrin from human milk' (L0520 Sigma-Aldrich) until a purity greater than 99% by SDS-PAGE was attained.

Immunisation of rabbits.

Immunisation is carried out intradermally (day 1), inoculating 250 µg of purified lactoferrin in 0.5 mL of PBS to which 0.5 mL of Freund's Complete Adjuvant is added. After the immunisation, a booster is administered every three weeks until a course of three boosters are given in total. 250 µg of antigen is administered intramuscularly in each dose of 0.5 mL total volume (to which 0.5 mL of Freund's Incomplete Adjuvant is added).

Two weeks after the administration of each booster bloodletting is performed to obtain the different serums. Blood is extracted from the auricular artery using a needle. Between 15 and 30 ml of whole blood are obtained from each bleeding (which is equivalent to 7 and 15 mL of serum). The serum is frozen until the subsequent purification.

Antibody purification by affinity.

A HiTrap™ NHS-activated HP 5 mL column (GE Healthcare) is prepared with 5 mg of purified lactoferrin according to the manufacturer's instructions. Pass 10 mL of hyperimmune rabbit serum through the aforementioned column and wash with PBS until the absorbance at 280 nm is less than 0.050. The antibodies are eluted in a 250 mM glycine buffer at pH 2.75 and the fractions are collected and immediately neutralised with phosphate buffer and then dialysed against PBS. The concentration is determined by its absorbance at 280 nm.

### Example 3. Preparation of the conjugates.

### Calprotectin

A conjugate of the CA1 antibody is prepared with polystyrene microparticles. The particles used are coloured, of 200 nm nominal diameter and with carboxyl groups on their surface (K1 020 brand Estapor, Merck, Darmstadt, Germany). 1 mL of 10% particles are washed by centrifugation and resuspended in 10 mM MES buffer (2-(N-morphilino)ethanosulphonic acid) at pH 6 to which EDC (1-ethyl-3-(3-dimethylaminopropyl)-carbodiimide) is added until a concentration of 5 mM is reached. It is incubated for 1 hr at 37 °C and the excess reagent is removed by centrifugation. The activated particles are resuspended in 10 mM MES at pH 6 and the anti-calprotectin monoclonal antibody is added up to a surface concentration of 2 mg/m², after which it is incubated for 18 hr at 4 °C before washing with 0.1% Tween 20.

### Lactoferrin

Similarly, a conjugate of an affinity purified anti-lactoferrin polyclonal antibody with polystyrene microparticles is prepared. The particles used are coloured, of 300 nm nominal diameter and with carboxyl groups on their surface (K1 030 brand Estapor, Merck, Darmstadt, Germany). Equally, 1 mL of 10% particles are washed by centrifuge and resuspended in 10 mM MES buffer (2-(N-morphilino)ethanosulphonic acid) at pH 6 to which EDC (1-ethyl-3-(3-dimethylaminopropyl)-carbodiimide) is added until a concentration of 5 mM is reached. It is incubated for 1 hr at 37 °C and the excess reagent is removed by centrifuge. The activated particles are resuspended in 10 mM MES at pH 6 and the affinity purified anti-lactoferrin polyclonal antibody is added up to a surface concentration of 1 mg/m², after which it is incubated for 4 hr at 37 °C before washing with 0.1% Tween 20.

The conjugates with particles for the control line are obtained in the same way but using streptavidin (S4762, Sigma-Aldrich) to a final surface concentration of 1 mg/m².

A mix of the three conjugates with said particles in concentrations of 0.02%, 0.04% and 0.05% respectively, are diluted in a solution of 10% sucrose, 2% bovine casein, 1% PEG-6000 and 2% Tween-20 with TRIS buffer (tris(hydroxymethyl)aminomethane) at pH 8. This solution is deposited at a ratio of 15 µL/cm in a wound material with 29 mm wide, non-interwoven polyester fibres which, after deposition, are dried in a 45 °C air current (for 5 minutes) and then for 24 hr in a chamber at 30°C and 20% relative humidity.

### Example 4. Preparation of the test and control lines.

The anti-lactoferrin, anti-calprotectin and anti-streptavidin (for the control line) antibodies are dialysed against PBS, taken to a concentration between 0.5 and 1 mg/mL and then deposited in parallel lines on a 25 mm wide, laminated nitrocellulose membrane (Millipore Hi-Flow Plus) of pore sizes between 10 and 30 microns at a ratio of 1 µL/cm. After the deposition, the membrane is dried in a 45 °C air current (for 2 minutes) and then immediately put into a chamber for 24 hr at 30°C and 20% relative humidity.

### Example 5. Setting up the immunochromatographic test.

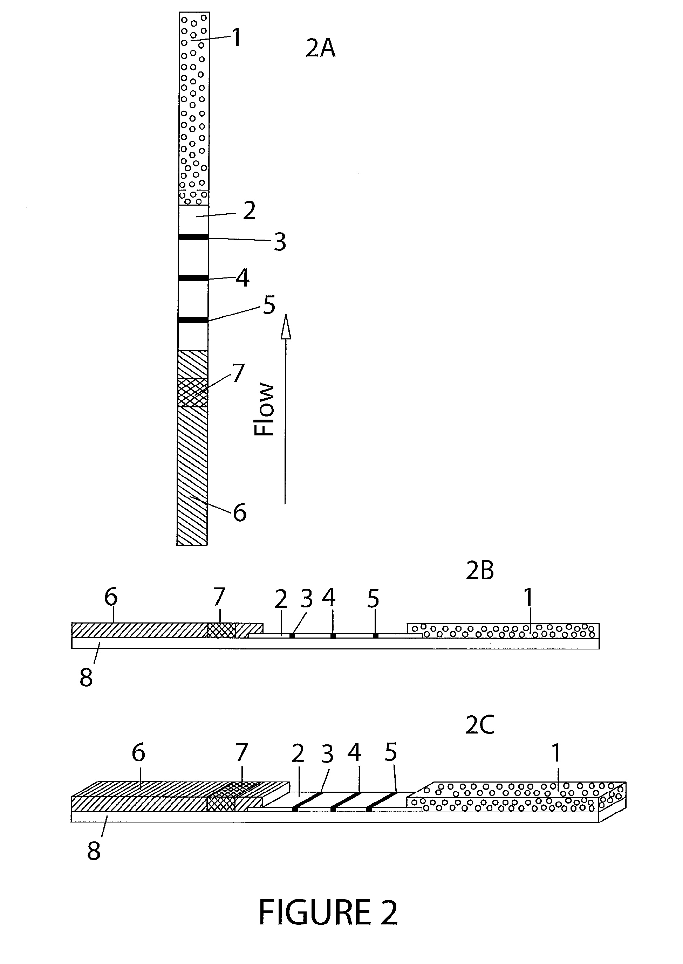

The material with the conjugate, the membrane and the absorbent material are mounted, as shown in Figure 2, over a plastic support with an adhesive lamina and the strips are then cut across the direction of their mounting at a width of 4 mm.

A solution for the dispersion of the faeces samples is prepared that consists of an aqueous solution of 300 mM sodium chloride, 1% Triton X-100, 100 µg/mL of non-specific mouse IgG antibodies and 200 µg/mL of non-specific rabbit IgG antibodies in 200 mM TRIS buffer at pH 9. The preparation is dispensed into sampling vials at a ratio of 1 mL/vial.

### Example 6. Faecal calprotectin and lactoferrin detection.

For the device of the invention to be suitable for diagnostic use, it is necessary to use some cut-off values, in other words, the analyte concentration for above which the test is positive and for below which the test is negative. In the case of calprotectin, the cut-off value is 50 µg hCp/g of faeces and for lactoferrin it is 10 µg hLf/g of faeces.

Due to the existence of the cut-off value, it is necessary to take faeces samples in a quantified manner. To do this a specific vial is used for stool sampling which contains a stem with slots for the sample, and a reducer to eliminate the excess sample, in such a way that the system has been calibrated for the introduction of exactly 10 mg of stool sample into the vial. Moreover, as the vial contains 1 mL of solvent, the sample concentration can be quantified.

### Calprotectin:

1/2 serial dilutions of calprotectin are prepared in the sample dispersion solvent described in the previous section. 100 µL of these dilutions are applied to the immunochromatographic devices of the invention and the test is left to develop for 10 minutes at room temperature (25 °C) after which the result can be interpreted by visual inspection of the appearance of the test line. The same operation is carried out but this time diluting the calprotectin in a sample pool of faeces resuspended in the same buffer at an approximate ratio of 1/100. The results are shown in Table 1.

**Table 1**

| ng hCp/mL | In solvent | In faeces | mg hCp/g faeces |
|---|---|---|---|
| 16000 | + | + | 1600 |
| 8000 | + | + | 800 |
| 4000 | + | + | 400 |
| 2000 | + | + | 200 |
| 1000 | + | + | 100 |
| 500 | + | + | 50 |
| 250 | - | - | 25 |
| 125 | - | - | 12.5 |
| 0 | - | - | 0 |

### Lactoferrin:

1/2 serial dilutions of lactoferrin are prepared in the sample dispersion solvent described in the previous section. 100 µL of these dilutions are applied to the immunochromatographic devices of the invention and the test is left to develop for 10 minutes at room temperature (25 °C) after which the result can be interpreted by visual inspection of the appearance of the test line. The same operation is carried out but this time diluting the lactoferrin in a sample pool of faeces resuspended in the same buffer at an approximate ratio of 1/100. The results are shown in Table 2.

**Table 2**

| ng hLf/mL | In solvent | In faeces | mg hLf/g faeces |
|---|---|---|---|
| 3200 | + | + | 320 |
| 1600 | + | + | 160 |
| 800 | + | + | 80 |
| 400 | + | + | 40 |
| 200 | + | + | 20 |
| 100 | + | + | 10 |
| 50 | - | - | 5 |
| 25 | - | - | 2.5 |
| 0 | - | - | 0 |

A stool sample that contains a human faecal calprotectin concentration of greater than or equal to 50 µg/g of faeces and a lactoferrin concentration of greater than or equal to 10 µg/g in faeces, gives positive results using the test of the present invention.

### Example 7. Evaluation of the test's performance.

The performance of the invention's diagnostic device was compared with immunoassays for both lactoferrin and calprotectin. A study was carried out with 64 stool samples taken from the Department of Microbiological Services of a local hospital (Zaragoza, Spain). Three types of assay were carried out with the said samples:
- the method of the present invention.
- the ELISA type immunoassay by Calprest^{®} (Eurospital Spa, Trieste, Italy), performed according to the manufacturer's instructions.
- the rapid ICT test IBD EZ VUE^{®} (TechLab, Blacksburg, VA, USA), performed according to the manufacturer's instructions.

Calprotectin detection with the test of the present invention demonstrated a correlation in sensitivity of >94% compared with the Calprest^{®} test (Eurospital). And the detection of human faecal lactoferrin using the test of the present invention demonstrated a correlation in sensitivity of >99% compared with the rapid test of IBD EZ VUE^{®} (TechLab).

## Claims

1. An immunochromatographic device for the analysis of stool samples **characterised in that** it comprises:
a. A results window of three lines, one a control, another for the detection of calprotectin and another for the detection of lactoferrin, in such a way that it permits stool calprotectin and lactoferrin to be simultaneously identified and differentiated.
b. A porous membrane on to which anti-calprotectin and anti-lactoferrin antibodies have been separately immobilised.
c. An absorbent on to which have been deposited conjugates of anti-calprotectin and anti-lactoferrin antibodies bound to marker molecules or markers.

2. An immunochromatographic device according to claim 1, **characterised in that** the antibodies used both on the membrane and in the conjugates are polyclonal or monoclonal.

3. A diagnostic method for inflammatory bowel disease (IBD) **characterised in that** it comprises:
a. The obtaining of a stool sample,
b. the dispersion of the stool sample material in a buffer or diluent,
c. the application of this dispersion on the sample window of the device in claim 1,
d. the interpretation of the result according to the appearance of bands in the results window.

4. A rapid screening method for infectious diarrhoea (ID) **characterised in that** it comprises the procedure of claim 3.

5. A method to aid in the diagnosis of colorectal cancer (CRC) **characterised in that** it comprises the procedure of claim 3.

6. A kit for carrying out the diagnostic methods of claims 3, 4 and 5 **characterised in that** it comprises :
a. An immunochromatographic device according to claim 1,
b. a suitable diluent for suspension and dispersion of the stool material,
c. a vial for taking and handling the sample.
